# EUROPEAN PATENT SPECIFICATION

(11) **EP 3 563 895 B1**
(45) Date of publication and mention of the grant of the patent: **25.11.2020**
(21) Application number: 18170438.8
(22) Date of filing: 02.05.2018
(51) Int. Cl.: A61M 16/06

(54) **RESPIRATORY MASK WITH A FLEXIBLE CUSHION DETACHABLY COUPLED TO THE MASK BODY**
ATEMMASKE MIT EINEM FLEXIBLEN KISSEN, DAS LÖSBAR AN DEN MASKENKÖRPER BEFESTIGT IST
MASQUE RESPIRATOIRE AVEC COUSSIN SOUPLE COUPLÉAMOVIBLE AU CORPS DE MASQUE

(43) Date of publication of application: 06.11.2019
(73) Proprietor: Air Liquide Medical Systems, 92160 Antony (FR)
(72) Inventor: ALBERICI, Luca, 25030 Roncadelle (IT); MASSERDOTTI, Fulvio, 25075 Nave (IT); SANDONI, Giuseppe, 25124 Brescia (IT)
(74) Representative: Pittis, Olivier

(56) References cited:
- EP-A1- 2 954 920
- EP-A1- 2 954 921
- US-A1- 2010 163 049

## Description

The invention concerns a respiratory mask, in particular a nasal mask, in particular for adult or pediatric uses in the treatment of respiratory conditions or diseases affecting adults, children, toddlers, infants or newborns. The respiratory mask comprises a flexible cushion coupled to a main body, and a coupling system that prevents any misalignment and wrong coupling of the cushion to the main body of the mask.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD), etc.

Nasal masks deliver a flow of breathable gas for, or to assist in, patient respiration. Such a respiratory mask commonly comprises a rigid or semi-rigid mask shell or body, usually made of polymer, coupled to a soft face-contacting flexible cushion that comes into contact with the patient's face and conforms to the various facial contours of the patient face. The flexible cushion and the mask body are in fluid communication.

In case of a nasal mask, the flexible cushion defines, alone or with the mask body, a breathing chamber that receives at least a part of the patient's nose. A respiratory gas, such as air, is introduced into said breathing chamber so that the patient can inhale it.

This face-contacting cushion is usually made of soft, resilient elastomeric material, such as soft silicone or similar material. Masks additionally may have a forehead support and a headgear for correctly positioning, maintaining and/or securing the mask on the head of a patient. An example of a nasal mask of this kind is given by EP-A-462701.

The mask assembly is secured to the wearer's head by straps or similar devices thereby forming a headgear that can be adjusted to pull the mask against the face with sufficient force to achieve a gas tight seal between the mask and the wearer's face as taught by EP-A-462701, EP-A-874667, EP-A-1972357 or WO-A-00/57942.

Further, US-A-2012/234326 teaches a nasal mask comprising a flexible cushion coupled to a mask frame. In some embodiments, the cushion comprises a first and a second tab that aid in aligning the frame and the cushion, and in preventing the frame from rotating relative to the cushion.

WO-A-2010/135785 teaches a mask having a complex architecture including a cushion, a mask body and an intermediary element for coupling the cushion to the mask body.

EP2954921 discloses a respiratory mask.

For cleaning or for replacing worn out parts, the patient has to regularly uncouple the mask body and the flexible cushion, and recouple them afterwards. However, while uncoupling those parts may be easy, it has been noticed in practice that re-coupling them is not obvious for the patients, especially for elderly people, and often results in a misalignment of the flexible cushion vis-a-vis the mask body, leading to an impaired gas tightness, i.e. gas leaks, and a discomfort for the patient, that may negatively impact the efficiency of the gas treatment provided to the patients.

Hence, **the problem to be solved** is to provide an improved respiratory mask architecture, especially a nasal mask, ensuring an easy alignment and securing of the flexible cushion to the mask body, thereby improving the comfort of use for the patients and the gas tightness (i.e. seal) in preventing the escape of gas that may result from a misalignment of the cushion with respect to the mask body.

**A solution according to the present invention** concerns a respiratory mask, in particular a nasal mask, comprising a mask body and a flexible cushion detachably attached, i.e. coupled to, to the mask body,
characterized in that:
- the mask body comprises a first and a second tubular structure, said first and second tubular structures being coaxially arranged and spaced apart from each other by a first annular lodging, wherein :
   - the first tubular structure has a first inner diameter (D1) and the second tubular structure has a second inner diameter (D2) with D1<D2,
   - and the second tubular structure comprises at least a notch,
- the flexible cushion comprises a third and a fourth tubular structure, said third and fourth tubular structures being coaxially arranged and spaced apart from each other by a second annular lodging, wherein :
   - the third tubular structure has a third inner diameter (D3) and the fourth tubular structure has a fourth inner diameter (D4) with D3<D4 and D4>D2>D3>D1,
   - and at least a protruding element is arranged in the second annular lodging,
and wherein, when the flexible cushion is attached to the mask body:
- the second tubular structure of the mask body is inserted into the second annular lodging of the flexible cushion and held therein, i.e. "sandwiched" and "squeezed", by the third and fourth tubular structures of the flexible cushion,
- the third tubular structure of the flexible cushion is inserted into the first annular lodging of the mask body and held therein, i.e. "sandwiched" and "squeezed", by the first and second tubular structures of the mask body, and
- the protruding element is at least partially lodged into the notch.

A respiratory mask according to the present invention can further comprise one or more of the following additional features:
- the protruding element is mostly or totally lodged, i.e. inserted, into the notch, when the cushion is attached to the mask body.
- the protruding element is projecting upwardly in the second annular lodging of the cushion.
- the protruding element and the notch have complementary shapes, i.e. the shape of the protruding element is matching the shape of the notch.
- the first and second tubular structures of the mask body and the third and fourth tubular structures of the flexible cushion form collar structures.
- the flexible cushion is firmly coupled to the mask body by means of the collar structures that cooperate together.
- the first tubular structure forms an inner collar in the mask body having a first height H1 and the second tubular structure forms an outer collar in the mask body having a second height H2, with H1 < H2.
- for example, H1 is of between 5 mm and 13 mm, and/or H2 is of between 7 mm and 15 mm (with H1 < H2).
- the third tubular structure forms an inner collar in the cushion having a third height H3 and the fourth tubular structure forms an outer collar in the cushion having a fourth height H4, with H4 < H3.
- for example, H3 is of between 7 mm and 13 mm, and/or H4 is of between 5 mm and 11 mm (with H4 < H3).
- the first and second tubular structures project from the rear face of the mask body, the rear face being the face of the mask body that is facing the cushion, when the mask body and the cushion are coupled together.
- the third and fourth tubular structures of the flexible cushion project from the front face of the cushion, the front face being the outer face of the cushion that is facing the mask body, i.e. the rear face of the mask body, when the mask body and the cushion are coupled together.
- the mask body comprises a first gas passage, also called central opening, the first and second tubular structures being coaxially arranged around said first gas passage.
- the flexible cushion comprises a second gas passage, the third and fourth tubular structures being coaxially arranged around said second gas passage.
- the first gas passage of the mask body is in fluid communication with the second gas passage of the flexible cushion, when the flexible cushion is attached to the mask body.
- the flexible cushion is snap-fit attached to the mask body.
- the first and second tubular structures of the mask body and the third and fourth tubular structures of the flexible cushion have circular sections and/or cylindrical shapes.
- the second tubular structure comprises a notch, i.e. a recess or a cut, arranged in the upper border of the mask body.
- the mask body comprises two lateral arms projecting laterally from the mask body.
- the mask body comprises an upper holding arm projecting upwardly from the mask body.
- the two lateral arms and holding arm are integrally fixed to the mask body.
- the flexible cushion is made of a soft material, preferably silicone.
- the first tubular structure of the mask body has a first inner diameter (D1) of between 20 mm and 23 mm.
- the second tubular structure of the mask body has a second inner diameter (D2) of between 27 mm and 30 mm.
- the third tubular structure of the cushion has a third inner diameter (D3) of between 24 mm and 27 mm.
- the fourth tubular structure of the cushion has a fourth inner diameter (D4) of between 30 mm and 33 mm.
- the mask is a nasal mask, i.e., in use, the cushion covers the nose of the patient.
- the protruding element forms a protuberance in the second annular lodging of the flexible cushion.
- the mask body is essentially formed by the first and second tubular structures that are coaxially coupled together.
- the two lateral arms and the holding arm are integral with the peripheral wall of the mask body, preferably formed in one-piece, such as molded in one piece.
- the mask body is made of (polymeric) plastic material.
- the tubular-shape portion forming the main body, the holding arm and of the two lateral arms are made of a plastic material.
- the holding arm and the two lateral arms are made of a flexible plastic material so as to be slightly bendable toward the patient's face under the forces applied by the headgear, when said headgear is adjusted to the morphology of the patient's head.
- the plastic material is chosen among polycarbonate (PC), polypropylene (PP) or nylon.
- the two lateral arms and the holding arm each comprise a proximal end integral with the mask body, and a free distal end.
- the two lateral arms and the holding arm each comprise headgear connections located at their free ends, such as slots, hooks or any other structure suitable for fixing the straps of a headgear thereto.
- the flexible cushion and protruding element are made of silicone or similar.
- a hollow curved connector is fixed to the mask body and rotatable with respect to said mask body.
- a hollow curved connector is inserted into to the first gas passage (i.e. central opening) of the mask body and detachably held therein.
- the mask further comprises a headgear comprising several straps connected to the headgear connections of the holding arm and of the two lateral arms. The headgear is used for maintaining and securing the mask in a desired position on the head of the patient and on at least a part of the facial region, in particular on the nasal region, of the patient. Preferably, the straps of the headgear are fixed to the free distal ends of the holding arm and/or of the two lateral arms. The straps are generally made of polymer or fabric material, or both.
- the holding arm and of the two lateral arms have an elongated shape, for example band or ribbon shapes. Other shapes are of course also possible.
- the holding arm has a length of about 2 to 12 cm, preferably of about 2 to 8 cm.
- each lateral arm has a length of about 3 to 10 cm, preferably of about 3 to 6 cm.
- the flexible cushion comprises an inner chamber, i.e. a respiratory chamber, in fluid communication with the gas passage, and a nose aperture in fluid communication with the inner chamber and adapted for receiving at least part of the patient's nose, when the patient wears the mask.
- the nose aperture of the flexible cushion comprises a peripheral border configured to ensure a gas tightness between the mask and the patient's face, in particular the nasal region.
- the flexible cushion comprises a peripheral border comprising one or several membranes arranged around along or part of the peripheral border, i.e. at the periphery of the nose aperture of the respiratory inner chamber. In other words, the membrane(s) form(s) a flexible skirt around the along or part of the periphery of the nose aperture of the respiratory chamber so as to ensure an efficient gas tightness while being in contact with the patient's face, when the latter wears the mask.
- the membrane(s) is (are) molded in one piece with the cushion body including the peripheral border.
- the membrane and the cushion are integral and formed by a single piece made of a resilient soft or semi-soft material, i.e. of silicone or a similar material.
- at least a region of the holding arm and of the two lateral arms is configured or shaped so as to match, the external profile, i.e. the outer contour, of the flexible cushion.
- the holding arm and/or the two lateral arms are configured to have a slightly curved-shape.
- the cushion has a general triangular, trapezoidal or saddle shape.
- the inlet orifice is arranged at the center of the cushion.
- the mask is a nasal mask and the cushion is configured and sized for coming into contact, when the mask is worn by the user or patient, with specific regions of the user or patient's face. Preferably, those regions comprise an intermediate nose region, an upper lip region and lateral nose regions, wherein:
   - the upper lip region is the area comprised between the nose and the upper lip;
   - the intermediate nose region is the area of the nose approximately located at the junction of bone and cartilage; and
   - the lateral nose regions are those located on each side of the nose.

The invention also concerns a respiratory assembly comprising a gas delivery device, such as a medical ventilator, and a respiratory mask according to the present invention, preferably the gas delivery device is connected to the respiratory mask by means of a gas line, such as a flexible hose. Typically, the gas line is directly or indirectly fluidly connected to the hollow curved connector.

An embodiment of a nasal respiratory mask according to the present invention is shown in the enclosed Figures, among which:
- Figure 1 represents a front view of an embodiment of a nasal mask according to the present invention,
- Figure 2 is a rear partial view of the mask body of the mask of Figure 1, and
- Figure 3 is a front view of the flexible cushion of Figure 1.

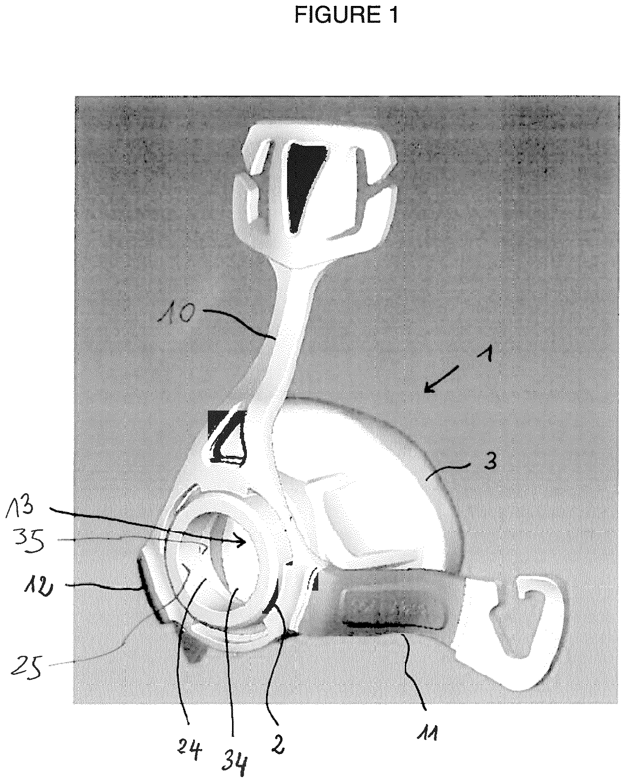

Figures 1-3 illustrate one embodiment of a nasal respiratory mask according to the present invention.

As shown in Figure 1, said nasal respiratory mask 1 comprises two main parts assembled together, namely a mask body 2 and a flexible cushion 3.

The mask body 2 has a simple and light structure as it is formed of a generally-cylindrical portion traversed by a central passage 24, and carrying two lateral arms 11, 12 and a holding arm 10 that are integrally fixed to the peripheral wall or surface of the mask body 2 as shown in Figure 1.

The mask body 2 can have a total width of between 80 and 110 mm, a total length of between 100 and 150 mm and its first gas passage 24 (central opening) has a diameter of about between 20 and 23 mm.

The holding arm 10 projects upwardly, i.e. about vertically, from said mask body 2, whereas the two lateral arms 11, 12 project laterally from said mask body 2 in opposite directions, i.e. one toward the right side of the mask 1 and the other toward the left side of the mask 1 as shown in Figure 1. The holding arm 10 further constitutes a frontal support, at its free end, that comes into contact with the patient's forehead (not shown), whereas the two opposite lateral arms or wings 11, 12 constitute right and left cheek supports (not shown), when the mask is worn by a patient. The holding arm 10 and the two opposite lateral arms 11, 12 are used for fixing a headgear thereto (not shown).

Preferably, the mask body 2, the two lateral arms 11, 12 and the holding arm 10 are molded in one piece. Typically, they are made of a polymer material that is slightly flexible, preferably a plastic material, such as polypropylene (PP), polycarbonate (PC) and nylon, or similar. Nevertheless, they can be made from several pieces or sub-units that are subsequently assembled together and made integral by means of any available fixing technique, for instance they can be glued, thermo-welded or similarly affixed to one another.

Further, the two lateral arms 11, 12 and the holding arm 10 can have lengths of about between 2 and 15 cm. For instance, the vertical axis of the holding arm 10 forms an angle of between 90 and 150° with the axis of each of the lateral arms 11, 12.

The two lateral arms 11, 12 and the holding arm 10 are integrally attached to the peripheral wall or surface of the mask body 2 by means of their proximal ends, whereas their free distal end comprising headgear connections, such as hooks or slots, for fixing thereto the straps of a headgear thereto (not shown) that is used for maintaining and securing the mask in a desired position on the head of the patient. Generally, the straps of headgears are made of polymer or fabric materials.

As shown in Figure 1, the two lateral arms 11, 12 and the holding arm 10 of the mask body 2 have elongated shapes, such as band or ribbon-like forms, and are slightly incurved so as to match the contours of the cushion 3, i.e. configured or shaped so as to conform to the external profile of the flexible cushion 3.

Furthermore, the mask 1 of the present invention also comprises a flexible cushion 3 that is fixed to the rear side 2a of the mask body 2. Typically, the flexible cushion 3 is a tridimensional hollow flexible structure forming a respiratory inner chamber 13 with a nose aperture adapted, i.e. conformed and sized, for receiving at least part of the patient's nose, when the patient wears the mask, so that the patient can breathe the respiratory gas contained in said inner chamber 13. The respiratory chamber 13 is in fluid communication with the first gas passage 24 of mask body 2, via a second gas passage 34, also called inlet orifice, arranged in the cushion 3 so that a respiratory gas flow can be provided to the respiratory chamber 13 and afterwards be inhaled by the patient. Typically, the second gas passage 34 is situated opposite to the nose aperture of the cushion 3.

Further, in order to provide efficient gas tightness (i.e. seal) and/or increased comfort for the patient, the nose aperture of the cushion 3 can be delimited by at least one flexible membrane that comes into contact with the patient's face and matches his/her facial morphology. The flexible membrane is arranged along all or part of the border of the nose aperture. The membrane(s) constitute(s) a kind of soft flexible skirt delimiting said the nose aperture of the cushion 3. Preferably, a unique or two superimposed membranes is/are used.

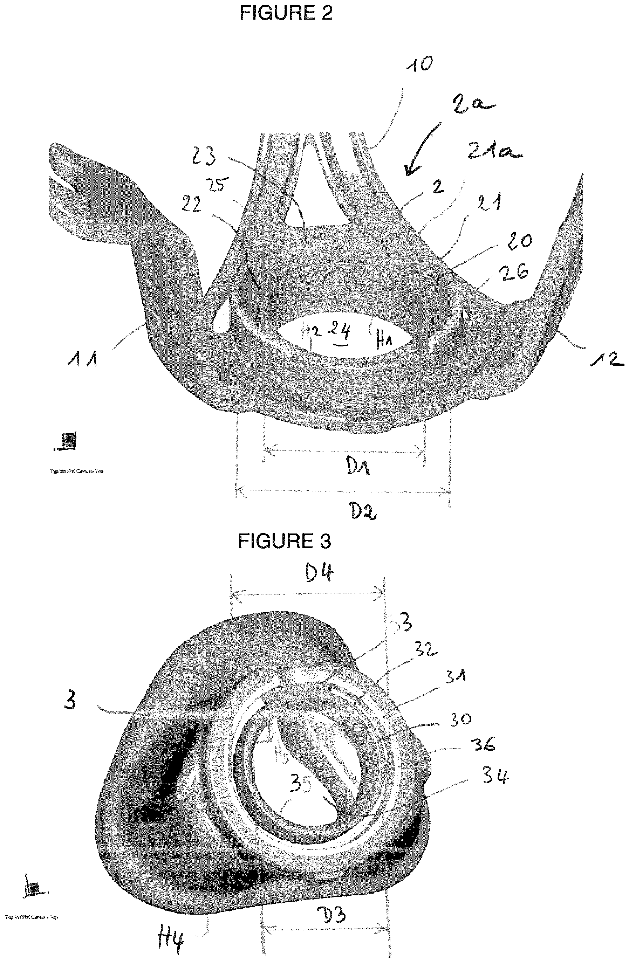

As shown in Figure 3, the cushion 3 has, in the present case, a generally trapezoidal or saddle tridimensional form or shape (rear view) so as to better match the contours of the patient's face, especially in the nasal regions. When the mask is worn by the patient, i.e. when the cushion 3 receives at least a part of the patient's nose, when said patient introduces his/her nose into the internal volume of the respiratory inner chamber 13 and breathes the gas contained therein, the cushion 3 and the membrane(s) arranged around the peripheral border of the nose aperture are in contact for example with the intermediate nose region (area at the junction of the bone and cartilage of the nose), the upper lip region (area between the nose and the upper lip), and the two opposite lateral regions of the nose of the patient (i.e. right and left flange regions of the nose). Of course, other embodiments and shapes are possible.

The cushion 3 is preferably made of a flexible and soft material, such as silicone or the like. Preferably, the membrane(s) and the rest of the cushion are molded in one piece and made of the same flexible soft material.

The flexible cushion 3 is detachably fixed to and held by the mask body 2 by means of a specific inter-connecting or coupling system as detailed below.

Indeed, according to the present invention, the mask body 2 comprises a first 20 and a second 21 tubular structure, such as walls forming collars as shown in Figure 2. The first and second tubular structures 20, 21 are coaxially arranged around the first gas passage 24 of the mask body 2 and spaced apart from each other by a first annular lodging 22. In other words, the first and second tubular structures 20, 21 are facing each other and separated by a spacing, a groove or similar, i.e. a small open space.

The first tubular structure 20 has a first inner diameter D1 and the second tubular structure 21 has a second inner diameter D2, such that D1<D2. For instance, D1 is of between 20 mm and 23 mm, whereas D2 is of between 27 mm and 30 mm.

The second tubular structure 21 comprises a notch 23, a notch, for instance a recess or a cut, as shown in Figure 2, arranged in its upper peripheral border 21a.

Similarly, the flexible cushion 3 comprises a third 30 and a fourth 31 tubular structure coaxially arranged around the second gas passage 34 of the flexible cushion 3 and spaced apart from each other by a second annular lodging 32, such as walls forming collars as shown in Figure 3. In other words, the third 30 and a fourth 31 tubular structures are also facing each other and also separated by a spacing, a groove or similar, i.e. a small open space.

The third tubular structure 30 has a third inner diameter D3 and the fourth tubular structure 31 has a fourth inner diameter D4 such that: D3<D4 and D4>D2>D3>D1.

For instance, D3 is of between 24 mm and 27 mm, whereas D4 is of between 30 mm and 33 mm.

Further, a protruding element 33 is arranged in the second annular lodging 32 of the flexible cushion 3.

Those first and second tubular structures 20, 21, third and a fourth tubular structure 30, 31, notch 23 and protruding element 33 are the main elements of the inter-connecting or coupling system allowing a detachable connection of the flexible cushion 3 to the mask body 2.

Indeed, when the flexible cushion 3 is attached to the mask body 2, the second tubular structure 21 of the mask body 2 is inserted into the second annular lodging 32 of the flexible cushion 3 and held therein by the third and fourth tubular structures 30, 31 of the flexible cushion 3, whereas the third tubular structure 30 of the flexible cushion 3 is inserted into the first annular lodging 22 of the mask body 2 and held therein by the first and second tubular structures 20, 21 of the mask body 2, and further the protruding element 33 is at least partially, preferably totally, lodged into the notch 23.

Preferably, the protruding element 33 and the notch 23 have (almost) complementary shapes.

Further, the first gas passage 24 of the mask body 2 is in fluid communication with the second gas passage 34 of the flexible cushion 3, when the flexible cushion 3 is attached to the mask body 2, i.e. when the first and second tubular structures 20, 21, cooperate with the third and a fourth tubular structure 30, 31, and the notch 23 cooperates with the protruding element 33 for holding the flexible cushion 3 secured to the mask body 2. In other words, the mask body 2 and the flexible cushion 3 are attached together by means of a snap-fit connection or the like.

Further, as the first and second tubular structures 20, 21 and the third and a fourth tubular structure 30, 31 are squeezed/sandwiched, as explained above and illustrated in the Figures, a gas tightness is ensured between them.

Preferably, the first and second tubular structures 20, 21 of the mask body 2 and the third and fourth tubular structures 30, 31 of the flexible cushion 3 have circular sections and cylindrical shapes, so as to constitute little collars that are interconnected and squeezed.

Preferably, the height H1 of the first tubular structure 20, i.e. inner collar 25 of the mask body 2, is less than the height H2 of the second tubular structure 21, i.e. outer collar 26 of the mask body 2, as shown in Figure 2. For instance, H1 is of between 5 mm and 13 mm, whereas H2 is of between 7 mm and 15 mm, with H1 < H2.

Likewise, the height H3 of the third tubular structure 30, i.e. inner collar of the cushion 3, is less than the height H4 of the fourth tubular structure 31, i.e. outer collar of the cushion 3, as shown in Figure 3. For instance, H3 is of between 7 mm and 13 mm, whereas H4 is of between 5 mm and 11 mm, with H4 < H3.

Generally speaking, the respiratory mask of the present invention, such as a nasal mask, is light and comfortable to wear.

The nasal respiratory mask of the present invention can be used in a method for treatment of a respiratory disorder or condition affecting infant, toddler and child patients as well as adult patients, for example in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Respiratory mask (1) comprising a mask body (2) and a flexible cushion (3) detachably attached to the mask body (2), **characterized in that**:
- the mask body (2) comprises a first and a second tubular structure (20, 21), said first and second tubular structures (20, 21) being coaxially arranged and spaced apart from each other by a first annular lodging (22), wherein :
• the first tubular structure (20) has a first inner diameter D1 and the second tubular structure (21) has a second inner diameter D2 with D1<D2,
• and the second tubular structure (21) comprises at least a notch (23),
- the flexible cushion (3) comprises a third and a fourth tubular structure (30, 31), said third and fourth tubular structures (30, 31) being coaxially arranged and spaced apart from each other by a second annular lodging (32), wherein :
• the third tubular structure (30) has a third inner diameter D3 and the fourth tubular structure (31) has a fourth inner diameter D4 with D3<D4 and D4>D2>D3>D1,
• and at least a protruding element (33) is arranged in the second annular lodging (32),
and wherein, when the flexible cushion (3) is attached to the mask body (2) :
- the second tubular structure (21) of the mask body (2) is inserted into the second annular lodging (32) of the flexible cushion (3) and held therein by the third and fourth tubular structures (30, 31) of the flexible cushion (3),
- the third tubular structure (30) of the flexible cushion (3) is inserted into the first annular lodging (22) of the mask body (2) and held therein by the first and second tubular structures (20, 21) of the mask body (2), and
- the protruding element (33) is at least partially lodged into the notch (23).

2. Respiratory mask according to Claim 1, **characterized in that** the mask body (2) comprises a first gas passage (24), the first and second tubular structures (20, 21) being coaxially arranged around the first gas passage (24).

3. Respiratory mask according to Claim 1, **characterized in that** the flexible cushion (3) comprises a second gas passage (34), the third and fourth tubular structures (30, 31) being coaxially arranged around the second gas passage (34).

4. Respiratory mask according to anyone of Claims 2 and 3, **characterized in that** the first gas passage (24) of the mask body (2) is in fluid communication with the second gas passage (34) of the flexible cushion (3), when the flexible cushion (3) is attached to the mask body (2).

5. Respiratory mask according to anyone of Claims 1 to 4, **characterized in that** the flexible cushion (3) is snap-fit attached to the mask body (2).

6. Respiratory mask according to anyone of Claims 1 to 5, **characterized in that** the first and second tubular structures (20, 21) of the mask body (2) and the third and fourth tubular structures (30, 31) of the flexible cushion (3) have circular sections and/or cylindrical shapes.

7. Respiratory mask according to anyone of Claims 1 to 6, **characterized in that** the notch (23) of the second tubular structure (21) is arranged in the upper border (21a) of the mask body (2).

8. Respiratory mask according to Claim 1, **characterized in that** the mask body (2) comprises two lateral arms (11, 12) projecting laterally from the mask body (2) and an upper arm (10) projecting upwardly from the mask body (2), said two lateral arms (11, 12) and holding arm (10) being integrally fixed to said main body (2).

9. Respiratory mask according to Claim 1, **characterized in that** the first and second tubular structures (20, 21) of the mask body (2) and the third and fourth tubular structures (30, 31) of the flexible cushion (3) form collar structures (25, 26; 35, 36).

10. Respiratory mask according to Claim 1, **characterized in that** the mask body (2) is made of polymeric material and/or the flexible cushion (3) is made of a soft material, preferably silicone.

11. Respiratory mask according to Claim 1, **characterized in that** the first inner diameter D1 of the first tubular structure (20) is between 20 mm and 23 mm and the second inner diameter D2 of the second tubular structure (21) is between 27 mm and 30 mm.

12. Respiratory mask according to Claim 1, **characterized in that** the thid inner diameter D3 of the third tubular structure (30) is between 24 mm and 27 mm and the fourth inner diameter D4 of the fourth tubular structure (31) is between 30 mm and 33 mm.

13. Respiratory mask according to Claim 1 or 9, **characterized in that** the first tubular structure (20) forms an inner collar (25) in the mask body (2) having a first height H1 and the second tubular structure (21) forms an outer collar (26) in the mask body (2) having a second height H2, with H1 < H2.

14. Respiratory mask according to Claim 1 or 9, **characterized in that** the third tubular structure (30) forms an inner collar (35) in the cushion (3) having a third height H3 and the fourth tubular structure (31) forms an outer collar (36) in the cushion (3) having a fourth height H4, with H4 < H3.

15. Respiratory mask according to Claim 1, **characterized in that** it is a nasal mask.

## Patentansprüche

1. Atemmaske (1), umfassend einen Maskenkörper (2) und ein flexibles Kissen (3), das abnehmbar an dem Maskenkörper (2) angebracht ist, **dadurch gekennzeichnet, dass**:
- der Maskenkörper (2) eine erste und eine zweite röhrenförmige Struktur (20, 21) umfasst, wobei die erste und die zweite röhrenförmige Struktur (20, 21) durch eine erste ringförmige Unterbringung (22) koaxial angeordnet und voneinander beabstandet sind, wobei:
• die erste röhrenförmige Struktur (20) einen ersten Innendurchmesser (D1) aufweist und die zweite röhrenförmige Struktur (21) einen zweiten Innendurchmesser (D2) aufweist, wobei D1<D2,
• und die zweite röhrenförmige Struktur (21) mindestens eine Kerbe (23) umfasst,
- das flexible Kissen (3) eine dritte und eine vierte röhrenförmige Struktur (30, 31) umfasst, wobei die dritte und die vierte röhrenförmige Struktur (30, 31) durch eine zweite ringförmige Unterbringung (32) koaxial angeordnet und voneinander beabstandet sind, wobei:
• die dritte röhrenförmige Struktur (30) einen dritten Innendurchmesser (D3) aufweist und die vierte röhrenförmige Struktur (31) einen vierten Innendurchmesser (D4) aufweist, wobei D3<D4 und D4>D2>D3>D1,
• und mindestens ein hervorstehendes Element (33) in der zweiten ringförmigen Unterbringung (32) angeordnet ist,
und wobei, wenn das flexible Kissen (3) an dem Maskenkörper (2) angebracht ist:
- die zweite röhrenförmige Struktur (21) des Maskenkörpers (2) in die zweite ringförmige Unterbringung (32) des flexiblen Kissens (3) eingeführt und darin von der dritten und der vierten röhrenförmigen Struktur (30, 31) des flexiblen Kissens (3) gehalten wird,
- die dritte röhrenförmige Struktur (30) des flexiblen Kissens (3) in die erste ringförmige Unterbringung (22) des Maskenkörpers (2) eingeführt und darin von der ersten und der zweiten röhrenförmigen Struktur (20, 21) des Maskenkörpers (2) gehalten wird, und
- das hervorstehende Element (33) mindestens teilweise in der Kerbe (23) untergebracht ist.

2. Atemmaske nach Anspruch 1, **dadurch gekennzeichnet, dass** der Maskenkörper (2) einen ersten Gasdurchgang (24) umfasst, wobei die erste und die zweite röhrenförmige Struktur (20, 21) koaxial um den ersten Gasdurchgang (24) angeordnet sind.

3. Atemmaske nach Anspruch 1, **dadurch gekennzeichnet, dass** das flexible Kissen (3) einen zweiten Gasdurchgang (34) umfasst, wobei die dritte und die vierte röhrenförmige Struktur (30, 31) koaxial um den zweiten Gasdurchgang (34) angeordnet sind.

4. Atemmaske nach einem der Ansprüche 2 und 3, **dadurch gekennzeichnet, dass** der erste Gasdurchgang (24) des Maskenkörpers (2) in Fluidverbindung mit dem zweiten Gasdurchgang (34) des flexiblen Kissens (3) steht, wenn das flexible Kissen (3) am Maskenkörper (2) angebracht ist.

5. Atemmaske nach einem der Ansprüche 1 bis 4, **dadurch gekennzeichnet, dass** das flexible Kissen (3) am Maskenkörper (2) durch Schnapppassung angebracht ist.

6. Atemmaske nach einem der Ansprüche 1 bis 5, **dadurch gekennzeichnet, dass** die erste und die zweite röhrenförmige Struktur (20, 21) des Maskenkörpers (2) und die dritte und die vierte röhrenförmige Struktur (30, 31) des flexiblen Kissens (3) kreisförmige Abschnitte und/oder zylindrische Formen aufweisen.

7. Atemmaske nach einem der Ansprüche 1 bis 6, **dadurch gekennzeichnet, dass** die Kerbe (23) der zweiten röhrenförmigen Struktur (21) im oberen Rand (21a) des Maskenkörpers (2) angeordnet ist.

8. Atemmaske nach Anspruch 1, **dadurch gekennzeichnet, dass** der Maskenkörper (2) zwei seitliche Arme (11, 12), die seitlich vom Maskenkörper (2) vorstehen, und einen Oberarm (10) umfasst, der vom Maskenkörper (2) nach oben vorsteht, wobei die beiden seitlichen Arme (11, 12) und der Haltearm (10) einstückig an dem Hauptkörper (2) befestigt sind.

9. Atemmaske nach Anspruch 1, **dadurch gekennzeichnet, dass** die erste und die zweite röhrenförmige Struktur (20, 21) des Maskenkörpers (2) und die dritte und die vierte röhrenförmige Struktur (30, 31) des flexiblen Kissens (3) Kragenstrukturen (25, 26; 35, 36) bilden.

10. Atemmaske nach Anspruch 1, **dadurch gekennzeichnet, dass** der Maskenkörper (2) aus Polymermaterial gefertigt ist und/oder das flexible Kissen (3) aus einem weichen Material, vorzugsweise Silikon gefertigt ist.

11. Atemmaske nach Anspruch 1, **dadurch gekennzeichnet, dass** der erste Innendurchmesser D1 der ersten röhrenförmigen Struktur (20) zwischen 20 mm und 23 mm und der zweite Innendurchmesser D2 der zweiten röhrenförmigen Struktur (21) zwischen 27 mm und 30 mm liegt.

12. Atemmaske nach Anspruch 1, **dadurch gekennzeichnet, dass** der dritte Innendurchmesser D3 der dritten röhrenförmigen Struktur (30) zwischen 24 mm und 27 mm und der vierte Innendurchmesser D4 der vierten röhrenförmigen Struktur (31) zwischen 30 mm und 33 mm liegt.

13. Atemmaske nach Anspruch 1 oder 9, **dadurch gekennzeichnet, dass** die erste röhrenförmige Struktur (20) einen inneren Kragen (25) in dem Maskenkörper (2), der eine erste Höhe H1 aufweist, bildet und die zweite röhrenförmige Struktur (21) einen äußeren Kragen (26) in dem Maskenkörper (2), der eine zweite Höhe H2 aufweist, bildet, wobei H1<H2.

14. Atemmaske nach Anspruch 1 oder 9, **dadurch gekennzeichnet, dass** die dritte röhrenförmige Struktur (30) einen inneren Kragen (35) in dem Kissen (3), der eine dritte Höhe H3 aufweist, bildet und die vierte röhrenförmige Struktur (31) einen äußeren Kragen (36) in dem Kissen (3), der eine vierte Höhe H4 aufweist, bildet, wobei H4<H3.

15. Atemmaske nach Anspruch 1, **dadurch gekennzeichnet, dass** es sich um eine Nasenmaske handelt.

## Revendications

1. Masque respiratoire (1) comprenant un corps de masque (2) et un coussinet souple (3) attaché de façon amovible au corps de masque (2), **caractérisé en ce que** :
- le corps de masque (2) comprend une première et une deuxième structure tubulaire (20, 21), lesdites première et deuxième structures tubulaires (20, 21) étant agencées de façon coaxiale et espacées l'une de l'autre par un premier logement annulaire (22), dans lequel :
• la première structure tubulaire (20) présente un premier diamètre intérieur D1 et la deuxième structure tubulaire (21) présente un deuxième diamètre intérieur D2, avec D1 < D2,
• et la deuxième structure tubulaire (21) comprend au moins une encoche (23),
- le coussinet souple (3) comprend une troisième et une quatrième structure tubulaire (30, 31), lesdites troisième et quatrième structures tubulaires (30, 31) étant agencées de façon coaxiale et espacées l'une de l'autre par un second logement annulaire (32), dans lequel :
• la troisième structure tubulaire (30) présente un troisième diamètre intérieur D3 et la quatrième structure tubulaire (31) présente un quatrième diamètre intérieur D4, avec D3 < D4 et D4 > D2 > D3 > D1,
• et au moins un élément saillant (33) est agencé dans le second logement annulaire (32),
et dans lequel, lorsque le coussinet souple (3) est attaché au corps de masque (2) :
- la deuxième structure tubulaire (21) du corps de masque (2) est insérée à l'intérieur du second logement annulaire (32) du coussinet souple (3) et maintenue en son sein par les troisième et quatrième structures tubulaires (30, 31) du coussinet souple (3),
- la troisième structure tubulaire (30) du coussinet souple (3) est insérée à l'intérieur du premier logement annulaire (22) du corps de masque (2) et maintenue en son sein par les première et deuxième structures tubulaires (20, 21) du corps de masque (2), et
- l'élément saillant (33) est au moins partiellement logé à l'intérieur de l'encoche (23).

2. Masque respiratoire selon la revendication 1, **caractérisé en ce que** le corps de masque (2) comprend un premier passage de gaz (24), les première et deuxième structures tubulaires (20, 21) étant agencées de façon coaxiale autour du premier passage de gaz (24).

3. Masque respiratoire selon la revendication 1, **caractérisé en ce que** le coussinet souple (3) comprend un second passage de gaz (34), les troisième et quatrième structures tubulaires (30, 31) étant agencées de façon coaxiale autour du second passage de gaz (34).

4. Masque respiratoire selon l'une quelconque des revendications 2 et 3, **caractérisé en ce que** le premier passage de gaz (24) du corps de masque (2) est en communication fluidique avec le second passage de gaz (34) du coussinet souple (3), lorsque le coussinet souple (3) est attaché au corps de masque (2).

5. Masque respiratoire selon l'une quelconque des revendications 1 à 4, **caractérisé en ce que** le coussinet souple (3) est attaché par emboîtement-pression au corps de masque (2).

6. Masque respiratoire selon l'une quelconque des revendications 1 à 5, **caractérisé en ce que** les première et deuxième structures tubulaires (20, 21) du corps de masque (2) et les troisième et quatrième structures tubulaires (30, 31) du coussinet souple (3) présentent des sections circulaires et/ou des formes cylindriques.

7. Masque respiratoire selon l'une quelconque des revendications 1 à 6, **caractérisé en ce que** l'encoche (23) de la deuxième structure tubulaire (21) est agencée dans la frontière supérieure (21a) du corps de masque (2).

8. Masque respiratoire selon la revendication 1, **caractérisé en ce que** le corps de masque (2) comprend deux bras latéraux (11, 12) dépassant latéralement du corps de masque (2) et un bras supérieur (10) dépassant vers le haut depuis le corps de masque (2), lesdits deux bras latéraux (11, 12) et bras de maintien (10) étant fixés d'un seul tenant audit corps principal (2).

9. Masque respiratoire selon la revendication 1, **caractérisé en ce que** les première et deuxième structures tubulaires (20, 21) du corps de masque (2) et les troisième et quatrième structures tubulaires (30, 31) du coussinet souple (3) forment des structures en collier (25, 26 ; 35, 36).

10. Masque respiratoire selon la revendication 1, **caractérisé en ce que** le corps de masque (2) est fait de matière polymère et/ou le coussinet souple (3) est fait d'une matière douce, de préférence de la silicone.

11. Masque respiratoire selon la revendication 1, **caractérisé en ce que** le premier diamètre intérieur D1 de la première structure tubulaire (20) fait entre 20 mm et 23 mm et le deuxième diamètre intérieur D2 de la deuxième structure tubulaire (21) fait entre 27 mm et 30 mm.

12. Masque respiratoire selon la revendication 1, **caractérisé en ce que** le troisième diamètre intérieur D3 de la troisième structure tubulaire (30) fait entre 24 mm et 27 mm et le quatrième diamètre intérieur D4 de la quatrième structure tubulaire (31) fait entre 30 mm et 33 mm.

13. Masque respiratoire selon la revendication 1 ou 9, **caractérisé en ce que** la première structure tubulaire (20) forme un collier intérieur (25) dans le corps de masque (2) présentant une première hauteur H1 et la deuxième structure tubulaire (21) forme un collier extérieur (26) dans le corps de masque (2) présentant une deuxième hauteur H2, avec H1 < H2.

14. Masque respiratoire selon la revendication 1 ou 9, **caractérisé en ce que** la troisième structure tubulaire (30) forme un collier intérieur (35) dans le coussinet (3) présentant une troisième hauteur H3 et la quatrième structure tubulaire (31) forme un collier extérieur (36) dans le coussinet (3) présentant une quatrième hauteur H4, avec H4 < H3.

15. Masque respiratoire selon la revendication 1, **caractérisé en ce qu'**il s'agit d'un masque nasal.
